# EUROPEAN PATENT APPLICATION

(11) **EP 4 039 284 A1**
(43) Date of publication of application: **10.08.2022**
(21) Application number: 22154474.5
(22) Date of filing: 01.02.2022
(51) Int. Cl.: A61L 2/08, B65B 9/10, B65B 9/06, B65B 55/24, B65B 55/08

(54) **PACKAGE PRODUCING MACHINE AND ASEPTIC CHAMBER FOR A PACKAGE PRODUCING MACHINE**

(30) Priority: 04.02.2021 EP 21155268
(71) Applicant: Tetra Laval Holdings & Finance S.A., 1009 Pully (CH)
(72) Inventor: APPARUTI, Daniele, 41051 Montale Rangone (IT); BARTOLINI, Robert, 41054 Marano sul Panaro (IT)
(74) Representative: Tetra Pak - Patent Attorneys SE

(57) **Abstract**

An aseptic chamber (100) of a package producing machine (10) is provided. The aseptic chamber comprises an entry section (110) for allowing a web of packaging material (20) to enter the aseptic chamber (100), and at least one electron beam emitter (152) arranged downstream the entry section (110) for treating the web of packaging material (20) as it runs through the aseptic chamber (100). The entry section (110) comprises an entry chamber (125) arranged between an infeed gasket (120) and an inner gasket (130).

## Description

### Technical Field

The invention relates to a package producing machine, i.e. to a machine being configured to form, fill, and seal individual packages. In particular, the present invention relates to an aseptic chamber for such package producing machine.

### Background Art

Within the food industry, beverages and other products are often packed in paper or paperboard based packages. Packages intended for liquid food are often produced from a packaging laminate comprising a core layer of paper or paperboard and an outer, liquid-tight layer of thermoplastic material on at least that side of the core layer which will form the inside of the packages.

One kind of packages are produced from so-called ready-to-fill packages. Such a ready-to-fill package is provided as a sleeve of packaging laminate like the one described above, being sealed at its bottom end prior to filling. Another type of ready-to-fill package is produced by providing an open-ended sleeve of a packaging laminate, and arranging, e.g. by injection molding, a plastic top at the upper end of the sleeve thereby leaving the bottom end open for filling. Another alternative for a package producing machine is to produce a series of consecutive packages from a web of packaging material, being formed to a tube-shape and being filled, sealed, and cut to separate the produced packages from the upstream tube.

In order to improve the package, the packaging material may be provided with an opening/closing means, such as a screw cap. This screw cap, being pre-applied to the packaging material and protruding from the otherwise planar surface of the packaging material, is thus treated by the various stations of the package producing machine before the final package is formed.

Before filling, the packaging material is sterilized or disinfected at least on the inside in order to extend the shelf-life of the product to be stored in the packages. Depending on the desired length of shelf-life, and depending on whether the packages are to be distributed and stored in a refrigerated environment or at room temperature, different levels of sterilization/disinfection are required. During recent years, systems for sterilization/disinfection using electron beam irradiation have reached the market. One or more electron beam emitters are arranged in an aseptic chamber which forms part of the package producing machine. The packaging material enters the aseptic chamber and passes the one or more electron beam emitters in order to be exposed to a sufficient dose of electrons. Once sterilized, the packaging material is ready to be filled with its desired content, formed, and sealed to form safe and hygienic packages.

During sterilization it is preferred to apply positive pressure inside the aseptic chamber and a ventilation system which is able to work with the positive pressure. This is required for safety purposes. For example, during sterilization, ozone is typically generated which should not be allowed to exit the aseptic chamber. In addition to this, at the entry section of the aseptic chamber emissions from the package producing machine may be drawn towards the electron beam emitters causing unwanted effects to the sterilization process, and possibly also causing defects to the packaging material. Such machine emissions may e.g. include water vapor, gas emissions or hot air.

While it has been possible to provide the required venting of the aseptic chamber, it is also important to provide proper seal of the entry section for the aseptic chamber. While this is normally a relatively easy task to solve for completely planar packaging material by using a suitable gasket, the inventors have realized that improvements are required when the packaging material is provided with locally protruding features, such as the above-described pre-applied closures.

Hence, there is a need for an improved aseptic chamber which prevents unwanted emissions to leak out, especially through the entry section of the aseptic chamber.

### Summary

It is an object of the invention to at least partly overcome one or more of the above-identified limitations of the prior art. In particular, it is an object to provide an aseptic chamber which reduces emissions to enter or exit the aseptic chamber.

According to a first aspect an aseptic chamber for a package producing machine is provided. The aseptic chamber comprises an entry section for allowing a web of packaging material to enter the aseptic chamber, and at least one electron beam emitter arranged downstream the entry section for treating the packaging material as it runs through the aseptic chamber. The entry section comprises an entry chamber arranged between an infeed gasket and an inner gasket.

The infeed gasket and/or the inner gasket may be provided with a cutout for allowing a pre-applied opening device of the packaging material to pass. The gaskets will thereby allow such pre-applied opening device, e.g. in the form of a screw cap, to pass without damaging the gaskets.

The width of the entry chamber may be dimensioned to accommodate the pre-applied opening device of the packaging material. As the pre-applied opening device fits inside the entry chamber, the infeed gasket and the inner gasket will never be open at the same time thereby improving sealing of the entry section.

The entry section may further comprise an exit chamber arranged between the inner gasket and an exit gasket. The entry section may thus be provided with three gaskets and two chambers, thereby improving the pressure inside the aseptic chamber and breaking the flow into the aseptic chamber.

The volume of the entry chamber may be larger than the volume of the exit chamber. Further reduction of the flow into the aseptic chamber from the entry section is thereby provided.

The exit gasket may be provided with a cutout for allowing a pre-applied opening device of the packaging material to pass. Also the exit gasket will thus allow the pre-applied opening device to pass without the risk for damaging the exit gasket.

The width of the exit chamber may be dimensioned to accommodate the pre-applied opening device of the packaging material. As the pre-applied opening device fits inside the exit chamber, the inner gasket and the exit gasket will never be open at the same time thereby improving sealing of the entry section.

The aseptic chamber may further comprise an evacuation pipe connected to the entry chamber. By evacuating the entry chamber, the possible risk for emissions to enter the aseptic chamber is further reduced.

The evacuation pipe may be connected to the entry chamber at a position below the infeed gasket and the inner gasket. When the packaging material is oriented such that the pre-applied opening device is directed downwards, evacuation of emissions surrounding the pre-applied opening device is improved.

The aseptic chamber may further comprise a venting pipe connected to the aseptic chamber downstream the entry section. Hence it is possible to maintain a controlled environment inside the aseptic chamber.

The evacuation pipe may be connected to the venting pipe by means of an Y-connector. This ensures no big flow detachments and recirculation zones, causing only as very small increase of turbulent kinetic energy.

According to a second aspect, a package producing machine is provided. The package producing machine comprises an aseptic chamber according to the first aspect.

According to a third aspect, a method for a package producing machine is provided. The method comprises: feeding a web of packaging material to an aseptic chamber, allowing the packaging material to enter an entry section of the aseptic chamber by passing an entry chamber arranged between an infeed gasket and a downstream inner gasket, and evacuating the entry chamber.

Still other objectives, features, aspects and advantages of the invention will appear from the following detailed description as well as from the drawings.

### Brief Description of the Drawings

Embodiments of the invention will now be described, by way of example, with reference to the accompanying schematic drawings, in which
Fig. 1 is a schematic view of a package producing machine according to an embodiment;
Fig. 2 is a cross-sectional view of an aseptic chamber according to an embodiment;
Fig. 3 is an isometric view of an entry section of an aseptic chamber according to an embodiment;
Figs. 4a-c are cross-sectional views of an entry section of an aseptic chamber according to an embodiment;
Fig. 4d is an isometric view in cross-section of the entry section shown in Figs. 4a-c; and
Fig. 5 is a schematic view of a method according to an embodiment.

### Detailed description

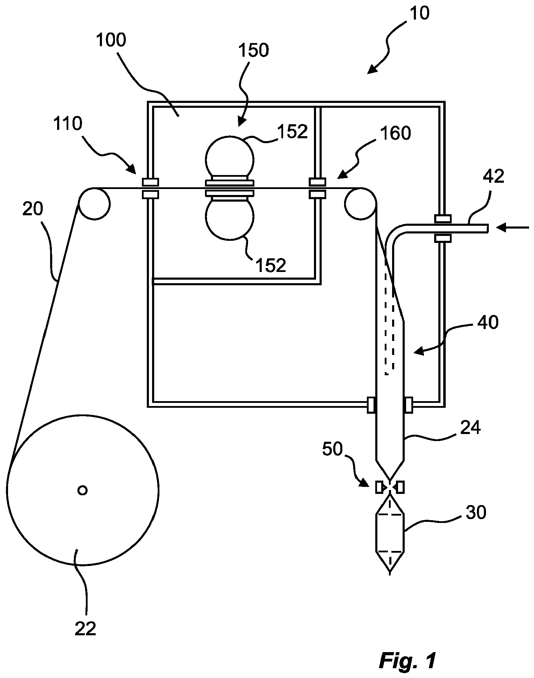

Starting in Fig. 1, a schematic view of the general principle of a package producing machine 10 is shown. As shown in Fig. 1, the package producing machine 10 is configured to transform a web of packaging material 20 to a series of individual packages 30.

The web of packaging material 20 is e.g. provided on a reel 22, and fed continuously through the package producing machine 10 where it passes several different stations. Although many different stations may be included in a package producing machine 10, the example of Fig. 1 specifically depicts an aseptic chamber 100, a filling station 40, and a sealing and forming station 50.

The web of packaging material 20 enters the aseptic chamber 100 through an entry section 110. It thereafter passes a sterilization unit 150 in the form of one or more electron beam emitters 152. After being exposed to electron beam irradiation, the web of packaging material 20 exits the aseptic chamber 100 through an exit section 160. In the filling station 40 the web of packaging material 20 is formed into a tube 24. The tube 24 is filled with the desired content, preferably a liquid food product, through a filling pipe 42 before the sealing and forming section 50 provides transversal seals to the tube 24, cuts the leading part of the tube 24 from the upstream tube 24 at the position of the transversal seal, and forms the separated package 30 to its desired shape.

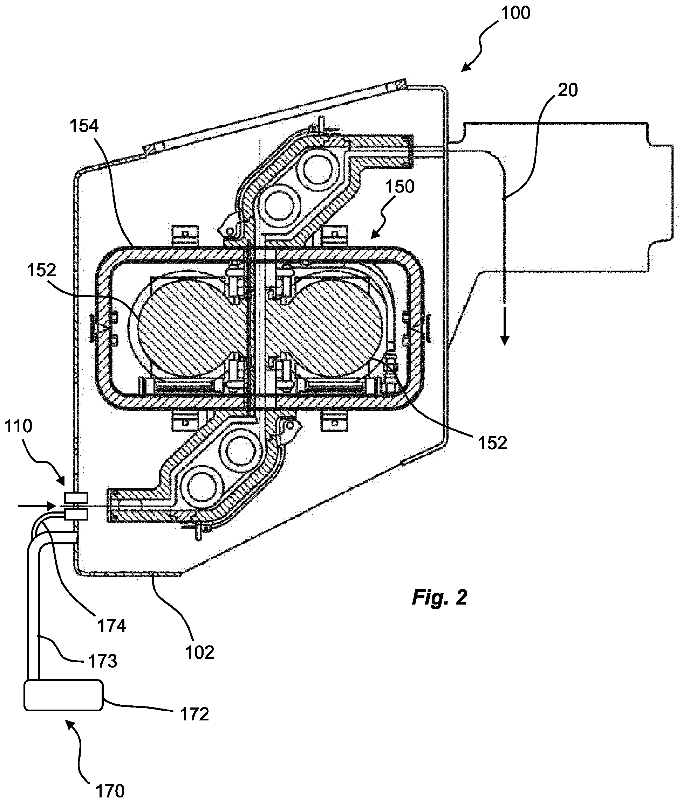

An embodiment of an aseptic chamber 100 is further shown in Fig. 2. The aseptic chamber 100 is enclosed by a housing 102. The entry section 110 (indicated schematically) is arranged at the housing 102, thereby allowing the web of packaging material 20 to enter the interior of the housing 102. The sterilization unit 150 is arranged inside the housing 102, and enclosed by a shield 154. In the shown example, two electron beam emitters 152 are arranged inside the shield 154 and arranged on opposite sides of the web of packaging material 20. Hence, when the web of packaging material 20 passes the sterilization unit 150 both sides of the web of packaging material 20 will be sterilized by electron beam irradiation.

The aseptic chamber 100 is provided with a venting system 170. The venting system 170 comprises a compressor 172 configured to control the environment inside the aseptic chamber 100. For this, the compressor 172 is connected to a venting pipe 173 which at its opposite end is connected to the housing 102. As will be explained further below, the venting system 170 further comprises an evacuation pipe 174 which is branched from the venting pipe 173 and connected to the entry section 110 of the aseptic chamber 100.

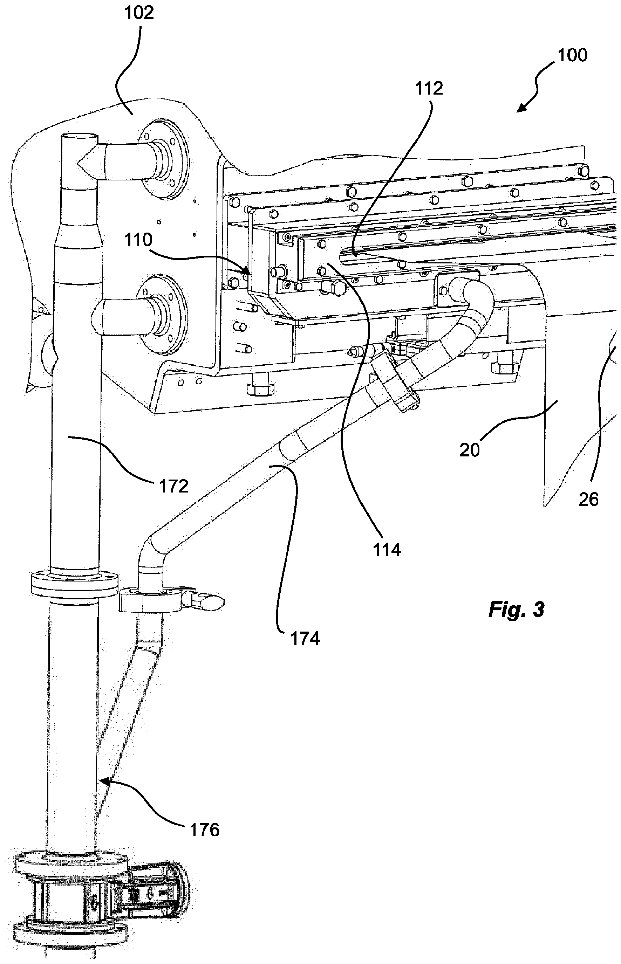

Now turning to Fig. 3, details of the entry section 110 of the aseptic chamber 100 are further shown. The web of packaging material 20 is, as previously explained, entering the aseptic chamber 100 through the entry section 110. Although shown only partly in Fig. 3, the web of packaging material 20 is preferably provided with pre-applied opening devices, such as screw caps 26. The opening devices 26 should be distributed on the web of packaging material 20 such that each formed package 30, when produced by an associated package producing machine 10, is provided with one, and only one, opening device 26.

The entry section 110 is provided with a slit 112 through which the web of packaging material 20 is fed. The slit 112 is formed in an exterior plate 114 and has a width dimensioned to allow the web of packaging material 20 to pass. Further, the height of the slit 112 is dimensioned such that any pre-applied opening device 26 of the packaging material can pass through.

The evacuation pipe 174 is connected at the center of the entry section 110, below the slit 112. As can be further seen in Fig. 3 the venting pipe 173 is connected to the housing 102 of the aseptic chamber 100, and the evacuation pipe 174 is connected to the venting pipe 173 by means of a Y-connection 176. Although not shown in Fig. 3, the Y-connection 176 is provided relatively close to the compressor 172, preferably the distance between the compressor 172 and the Y-connector 176 is less than the length of the evacuation pipe 174.

The central connection of the evacuation pipe 174 to the entry section 110 is particular advantageous for webs of packaging material 20 where the pre-applied opening device 26 is arranged centrally. In such embodiment the evacuation port is aligned with the pre-applied opening 26.

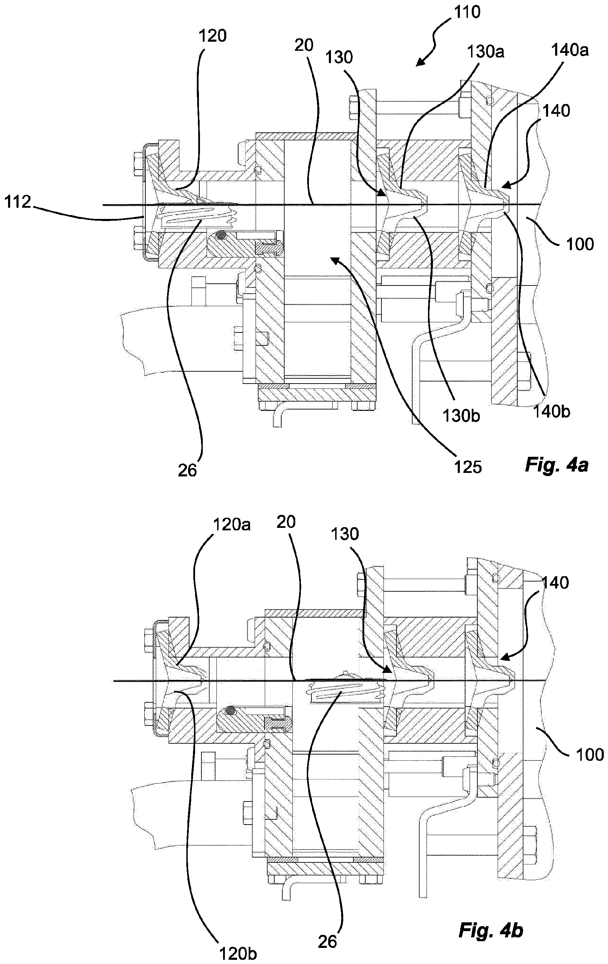

In Figs. 4a-c the entry section 110 is shown in cross-section together with a web of packaging material 20. In these figures the web of packaging material 20 is provided with pre-applied opening devices 26 in the form of threaded spouts extending away from the otherwise planar surface of the web of packaging material 20. The web of packaging material 20 is oriented such that the threaded spout 26 is directed downwards.

For the sake of clarity it should be noted that the threaded spout 26 is typically provided with a cap or closure before filling. Optionally, the threaded spout 26 extends from a sealed membrane of the web of packaging material 20 such that the cap or closure can be added after filling.

Starting in Fig. 4a, the web of packaging material 20 is positioned relative the entry section 110 such that the threaded spout 26 has passed the slit 112. Immediately inside the slit 112 an infeed gasket 120 is provided. The infeed gasket 120, which typically is provided as a pair of vertically aligned infeed gaskets 120a-b (see Fig. 4b), is in Fig. 4a partly deflected inwards due to the web of packaging material 20, and especially the threaded spout 26, pushing the infeed gasket 120 in the direction of the movement of the web of packaging material 20.

The infeed gasket 120 forms an upstream boundary for an entry chamber 125. The entry chamber 125 is sealed downstream by an inner gasket 130. The inner gasket 130 is also preferably formed as a pair of vertically aligned inner gaskets 130a-b. The presence of the web of packaging material 20 causes a small separation between the pair of inner gaskets 130a-b, which separation will increase as the pre-applied opening device 26 comes into contact with the inner gasket 130 and is forced through the gasket 130.

While the inner gasket 130 forms a downstream boundary for the entry chamber 125, the inner gasket 130 also forms an upstream boundary for an exit chamber 135. The exit chamber 135 extends in the machine direction between the inner gasket 130 and an exit gasket 140. The exit gasket 140 is preferably identical to the inner gasket 130 and/or the infeed gasket 120, i.e. formed as a pair of vertically aligned exit gaskets 140a-b.

Hence, when the web of packaging material 20 is running through the package producing machine 10, it will enter the entry section 110 and pass through the entry chamber 125 and the exit chamber 135 before it proceeds further into the aseptic chamber 100.

In Fig. 4b, the web of packaging material 20 has moved such that the opening device 26 is positioned at the downstream end of the entry chamber 125, immediately before reaching the inner gasket 130. At this position both the infeed gasket 120 and the inner gasket 130 are closed, sealing the entry chamber 125. As the evacuation pipe 174 is constantly providing suction of air from the entry chamber 125 any emissions carried by the opening device 26 and the adjacent web of packaging material 20 will be effectively removed before the opening device 26 reaches the interior of the aseptic chamber 100.

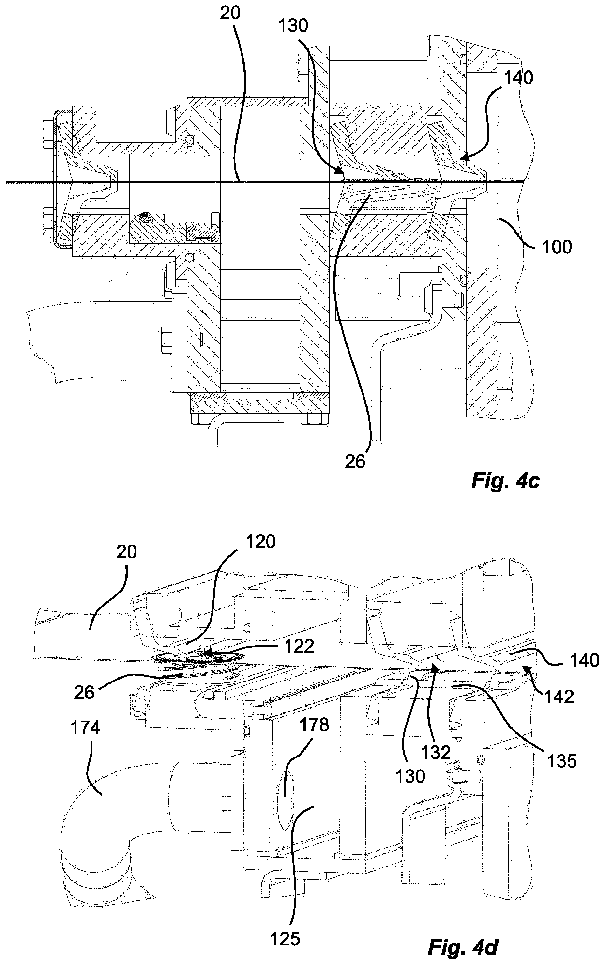

In Fig. 4c a further downstream position of the opening device 26 is shown, as it moves towards the interior of the aseptic chamber 100. A this position the opening device 26 has reached the inner gasket 130, forcing it to open in order to allow the opening device 26 to pass and enter the exit chamber 135, While the inner gasket 130 is partly open, as indicated in Fig. 4c, emissions from the interior of the aseptic chamber 100 are prevented to leak out due to the provision of the exit gasket 140. The exit gasket 140 is fully closed, sealing the boundary between the interior of the aseptic chamber 100 from the environment outside the entry section 110.

The distance between the inner gasket 130 and the exit gasket 140 is, as indicated in Figs. 4a-c, greater than the width of the opening device 26. This means that the opening device 26 will only cause one of the inner gasket 130 and the exit gasket 140 to open, while the other is closed. Hence, from the situation shown in Fig. 4c the inner gasket 130 will be allowed to close completely before the opening device 26 reaches the exit gasket 140, thereby forcing it to open.

The provision of the entry chamber 125 and the exit chamber 135, as well as the infeed sealing 120, the inner sealing 130, and the exit sealing 140 achieves a very well controlled environment for the opening device 26 as it passes through the entry section 110. The entry chamber 125 allows for evacuation of any emissions brought into the entry section 110 by the opening device 26, while the exit chamber 135 prevents those emissions to be transported further into the aseptic chamber. At the same time the exit chamber 135 will prevent any emissions from inside the aseptic chamber 100 to reach the outer environment.

The entry section 110 is further shown in Fig. 4d. The evacuation pipe 174 terminates at an evacuation port 178 of the entry chamber 125. The evacuation port 178 is arranged on the same side of the web of packaging material 20 as the opening device 26. However, the width of the entry chamber 125, as well as the width of the exit chamber 135, is preferably greater than the width of the web of packaging material 20 such that air is evacuated from the entire entry chamber 125 and exit chamber 135, respectively. As is further indicated in Fig. 4d, each gasket 120, 130, 140 is provided with a cut-out 122, 132, 142 for permitting pre-applied opening 26 passage and thereby avoiding the risk of mechanical impact and damage. While the necessity of having this cut-out 122, 132, 142 increases machine emissions during different phases of machine run, the provision of the entry chamber 125, the exit chamber 135, and the evacuation pipe 174 reduces such emissions drastically.

The entry section 110 provides for an improved solution related to the web of packaging material 20 entering the aseptic chamber 100. The concept of the entry section 110 described above has proven to reduce emissions by about 50% Particular advantages are due to the Y-connection 176 of the evacuation pipe 174 to the compressor 172 in order to increase evacuation inside the entry chamber 125. Further, by allowing the evacuation pipe 174 to connect to the entry chamber 125 using a funnel shape will intercept air flow produced by the cut-out 122 of the infeed gasket 120. Yet further, the presence of three gaskets 120, 130, 140 inside the entry section 110 with a total distance being smaller than the package repeat length will obstruct opening of the gaskets 120, 130, 140 for three times during the production of each package.

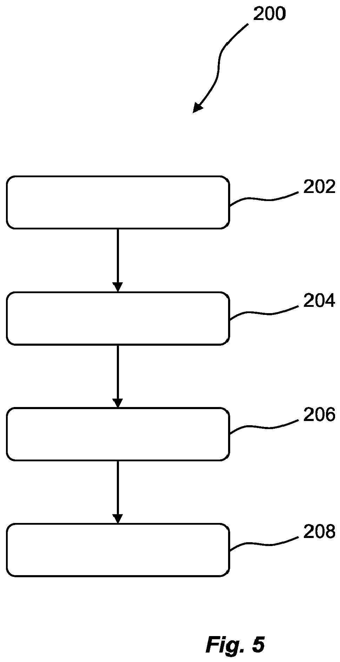

Now turning to Fig. 5, a method 200 for a package producing machine is schematically shown. The method 200 comprises a step 202 of feeding a web of packaging material 20 towards an aseptic chamber 100, and a step 204 of allowing the web of packaging material 20 to enter an entry section 110 of the aseptic chamber 100 by passing an entry chamber 125 arranged between an infeed gasket 120 and a downstream inner gasket 130. The method 200 further comprises a step 206 of evacuating the entry chamber 125.

Optionally, the method 200 may further comprise a step 208 of transporting the web of packaging material 20 through an exit chamber 135 arranged downstream the entry chamber 125, before reaching the interior of the aseptic chamber 100.

From the description above follows that, although various embodiments of the invention have been described and shown, the invention is not restricted thereto, but may also be embodied in other ways within the scope of the subject-matter defined in the following claims.

## Claims

1. An aseptic chamber (100) of a package producing machine (10), comprising an entry section (110) for allowing a web of packaging material (20) to enter the aseptic chamber (100), and at least one electron beam emitter (152) arranged downstream the entry section (110) for treating the web of packaging material (20) as it runs through the aseptic chamber (100),
wherein the entry section (110) comprises an entry chamber (125) arranged between an infeed gasket (120) and an inner gasket (130).

2. The aseptic chamber (100) according to claim 1, wherein the infeed gasket (120) and/or the inner gasket (130) is provided with a cutout (122, 132) for allowing a pre-applied opening device (26) of the web of packaging material (20) to pass.

3. The aseptic chamber (100) according to claim 2, wherein the width of the entry chamber (125) is dimensioned to accommodate the pre-applied opening device (20) of the web of packaging material (20).

4. The aseptic chamber (100) according to any of claims 1 to 3, wherein the entry section (110) further comprises an exit chamber (135) arranged between the inner gasket (130) and an exit gasket (140).

5. The aseptic chamber (100) according to claim 4, wherein the volume of the entry chamber (125) is larger than the volume of the exit chamber (135).

6. The aseptic chamber (100) according to claim 3 or 4, wherein the exit gasket (140) is provided with a cutout (142) for allowing a pre-applied opening device (26) of the web of packaging material (20) to pass.

7. The aseptic chamber (100) according to any of claims 4 to 6, wherein the width of the exit chamber (135) is dimensioned to accommodate the pre-applied opening device (26) of the web of packaging material (20).

8. The aseptic chamber (100) according to any of the preceding claims, further comprising an evacuation pipe (174) connected to the entry chamber (125).

9. The aseptic chamber (100) according to claim 8, wherein the evacuation pipe (174) is connected to the entry chamber (125) at a position below the infeed gasket (120) and the inner gasket (130).

10. The aseptic chamber (100) according to claim 8 or 9, further comprising a venting pipe (173) connected to the aseptic chamber (100) downstream the entry section (110).

11. The aseptic chamber (100) according to claim 8 or 9 and 10, wherein the evacuation pipe (174) is connected to the venting pipe (173) by means of an Y-connector (176).

12. A package producing machine (10), comprising an aseptic chamber (100) according to any of the preceding claims.

13. A method for a package producing machine, comprising: feeding a web of packaging material to an aseptic chamber, allowing the packaging material to enter an entry section of the aseptic chamber by passing an entry chamber arranged between an infeed gasket and a downstream inner gasket, and evacuating the entry chamber.
